(19) Europäisches Patentamt
European Patent Office
Office européen des brevets

(11) EP 2 878 676 A2

(12) **EUROPEAN PATENT APPLICATION**
published in accordance with Art. 153(4) EPC

(43) Date of publication:
**03.06.2015 Bulletin 2015/23**

(21) Application number: **13823422.4**

(22) Date of filing: **26.09.2013**

(51) Int Cl.:
***C12P 23/00*** $^{(2006.01)}$ ***C12N 1/12*** $^{(2006.01)}$

(86) International application number:
**PCT/CN2013/084262**

(87) International publication number:
**WO 2014/015841 (30.01.2014 Gazette 2014/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.07.2012 CN 201210264946**

(71) Applicants:
- **East China University Of Science And Technology
Shanghai 200237 (CN)**
- **Jiaxing Zeyuan Bio-Products Co., Ltd.
Jiaxin, Zhejiang 314006 (CN)**
- **Shanghai Zeyuan Marine Biotechnology Co. Ltd.
Shanghai 200237 (CN)**

(72) Inventors:
- **LI, Yuanguang
Shanghai 200237 (CN)**
- **ZHANG, Zhen
Shanghai 200237 (CN)**
- **FAN, Jianhua
Shanghai 200237 (CN)**
- **WAN, Minxi
Shanghai 200237 (CN)**
- **HOU, Dongmei
Shanghai 200237 (CN)**
- **ZHANG, Jingkui
Shanghai 200237 (CN)**
- **HUANG, Jianke
Shanghai 200237 (CN)**
- **LIANG, Songtao
Shanghai 200237 (CN)**
- **WANG, Jun
Shanghai 200237 (CN)**
- **CHEN, Jie
Shanghai 200237 (CN)**
- **WANG, Weiliang
Shanghai 200237 (CN)**
- **WANG, Jun
Shanghai 200237 (CN)**
- **LI, Shulan
Shanghai 200237 (CN)**
- **SHEN, Guomin
Shanghai 200237 (CN)**

(74) Representative: **Krauss, Jan
Boehmert & Boehmert
Anwaltspartnerschaft mbB
Patentanwälte Rechtsanwälte
Pettenkoferstrasse 20-22
80336 München (DE)**

# (54) METHOD USING MICRO-ALGAE FOR HIGH-EFFICIENCY PRODUCTION OF ASTAXANTHIN

(57) The present invention relates to a novel method for producing astaxanthin by using microalgae. The method comprises: heterotrophic cultivation of microalgae, dilution, photo-induction, collection of microalgal cells, and extraction of astaxanthin. The method according to the present invention takes full advantages of rapid growth rate in the heterotrophic stage and fast accumulation of astaxanthin in the photo-induction stage by using a large amount of microalgal cells obtained in the heterotrophic cultivation stage, so as to greatly improve the astaxanthin production rate and thereby achieve low cost, high efficiency, large scale production of astaxanthin by using microalgae. The method not only provides an important technical means to address the large scale industrial production of astaxanthin through microalgae but also ensures an ample source of raw material for the widespread utilization of astaxanthin.

EP 2 878 676 A2

## Description

### FIELD OF THE INVENTION

The present invention relates to microalgal biotechnology and particularly relates to a novel method for producing astaxanthin by using microalgae.

### BACKGROUND

Astaxanthin (chemical name: 3, 3 '- two - 4, 4' - 2 keto - beta, beta carotene, formula: $C_{40}H_{52}O_4$, relative molecular mass: 596.86, also known as xanthine shrimp, shrimp flavin pigment or lobster shell) is keto-carotenoid. It is in pink color, soluble in fat, insoluble in water, and soluble in organic solvents e.g. chloroform, acetone, benzene and carbon disulfideetc. In terms of chemical structure, astaxanthin comprises four isoprene units connected with conjugated double bond type links and two different pentene units at both ends, resulting in a six section ring structure as shown below. Because astaxanthin has the chemical structure comprising a long conjugated unsaturated double bond system, it is vulnerable to light, heat, and oxides so as to suffer from the destruction of its structure.

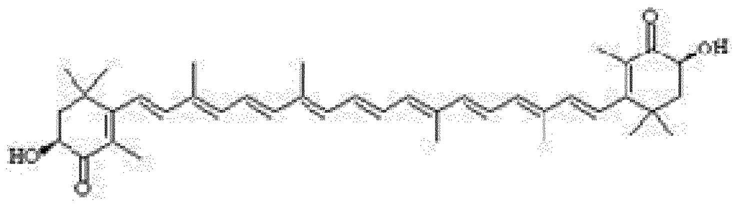

Astaxanthin is one kind of carotenoids and is also the highest level product in the carotenoid synthesis. Beta-carotene, lutein, Angle flavin, and lycopene are intermediate products in the carotenoid synthesis. Therefore, in nature, astaxanthin has the strongest antioxidant activity. So far the natural astaxanthin has been the strongest antioxidant compared to other existing antioxidants in nature, and thus the natural astaxanthin is well-known as "super antioxidant". Astaxanthin has an extensive application value not only for aquaculture feed additives and human food additives but also for medical, cosmetic, and health care products etc.

In comparison, using microalgae to produce astaxanthin has obvious advantages. Haematococcus which contains about 1-5% astaxanthin, based on cell dry weight, is the species with the highest astaxanthin content in nature. Firstly, astaxanthin in microalgae is mainly in the form of monoester and has a trans structure, the bioavailability of which is higher than chemically-synthesized astaxanthin which has a cis structure; Secondly, microalgae growth life cycle is short, its production equipment cover a small area, and its production is relatively stable in product quality and output; Finally, microalga (such as Haematococcus) is a product with high value, and contains a lot of protein, fat, polysaccharides and other active ingredients, and thus the comprehensive utilization of microalgae biomass can be realized by extracting and separating these active ingredients.

So far, production of astaxanthin from microalgae is mainly involved in photoautotrophic cultivation and heterotrophic cultivation.

The photoautotrophic cultivation of microalgae has disadvantages of slow growth rate, low cell density and low astaxanthin production rate. Up to date, the maximum cell dry weight in the photoautotrophic cultivation of microalgae was 6.8 g/L obtained in a 16 L bubble column bioreactor by Ranjbar et al. (cell production rate is 0.2 g/L/d) (Ranjbar R, Inoue R, Shiraishi H, Katsuda T, Katoh S: High efficiency production of astaxanthin by autotrophic cultivation of Haematococcus pluvialis in a bubble column photobioreactor. Biochemical Engineering Journal, 2008, 39 (3) 6:575-580.). The highest volumetric production rate of astaxanthin was 23.04 mg/L/d obtained in a 1 L photo-bioreactor by cultivation of *Haematococcus pluvialis* by Ranjbar (Ranjbar R, Inoue R, Katsuda T, Yamaji H, Katoh S: High efficiency production of astaxanthin in an airlift photobioreactor. Journal of Bioscience and Bioengineering 2008, 106(2):204-207.). The highest areal production rate of astaxanthin was 390 mg/m$^2$/d obtained in a 25000 L photo bio-reactor by outdoor cultivation of *Haematococcus pluvialis* by Olaizola while its cell production rate was only 0.052 g/L/d (Olaizola M: Commercial production of astaxanthin from Haematococcus pluvialis using 25,000-liter outdoor photobioreactors. Journal of Applied Phycology 2000, 12(3):499-506.). In addition, in the literature, the highest cell production rate reported by Garcia - Malea MC was 0.58 g/L/d for indoors culture in a 1.8 L bubble column reactor and 0.68 g/L/d for outdoor culture in a 220 L tubular reactor (airlift type) under such conditions of continuous culture which is not suitable for astaxanthin accumulation (Garcia-Malea MC , Acién FG, Fernández JM, Cerón MC, Molina E: Continuous production of green cells of Haematococcus pluvialis: Modeling of the irradiance effect. Enzyme and Microbial Technology 2006, 38(7):981-989.).

At present, according to the life cycle and morphological changes in the photoautotrophic cultivation of *Hae-*

*matococcus pluvialis,* the cultivation process is divided into two stages. The first stage (microalgae cultivation stage) is to cultivate *Haematococcus pluvialis* for the biomass rapid growth, the object of which is to obtain high cell density and high growth rate. To the best of our knowledge, there are two modes, namely, continues and semi-continuous cultivation. Continuous cultivation refers to microalgae cultivation under stable conditions to maintain the growth of *Haematococcus pluvialis* at a nearly constant growth rate and maintain the continuous production under a stable condition of physiological characteristics. Semi-continuous culture refers to microalgae cultivation wherein a portion of microalgae culture is transferred to stress environment every day after reaching a certain concentration, while the same amount of new culture is added for further cultivation. The second stage (astaxanthin accumulation stage) is to prompt the *Haematococcus pluvialis* to form cyst, subsequently to achieve the purpose of astaxanthin accumulation by the control means such as high light, high temperature, high salt, nutrient starvation and a series of stress. In these two different stages, the nutrition and environmental conditions are different. The current studies of interest here and aboard are mainly concentrated on selection and control of the conditions, and influences of the environmental factors etc. in the two stages. Normally, the first stage of photoautotrophic cultivation is aimed at increasing the cell number and density rather than accumulating the astaxanthin. At the late exponential phase (cell density reaches 0.5-1.5 g/L, the cell number reaches 0.2-0.5 $\times 10^6$ / ml at this time), because nitrogen and phosphorus nutrients are consumed, the cell culture without any operations e.g. dilution is directly transferred to the second stage at the same time, accompanied by exposure to high light, high temperature, high salinity and addition of culture medium with few nitrogen and phosphorus, for astaxanthin accumulation. At this stage, the cell number does not increase, or even decreases under the stress conditions sometimes. Due to spore and enlargement of cells, cell dry weight increases slowly by 2-4 times, i.e. up to 2-3 g/L at the end of the second stage compared to the beginning. Photoautotrophic medium and light requirement are different during the astaxanthin accumulation stage and the microalgae cultivation stage. The latter needs rich N, P content and requires reasonable proportion between each element (C, N, P, S, Na, Ca, K, Mg etc.). The former only requires a few salt materials, such as calcium salt, but normally without nitrogen and phosphorus.

Physical environment factors that affect the photoautotrophic cultivation of *Haematococcus pluvialis* include temperature, light intensity, pH value, dissolved oxygen and nutrient content etc. In literatures, there are quite a lot of reports, as shown in table 1.

Table 1 requirements of environmental factor at different stages

| Stage | Light(klx) | Temperature °C | pH | nutrient | | |
|---|---|---|---|---|---|---|
| | | | | C(mmol) | N(mmol) | P(mmol) |
| Vegetative cell cultivation stage | 1.1~2.0 | 15~25 | 7~8 | 10~30 | 2.5~10 | 0.5 |
| Cyst forming and astaxanthin accumulation stage | 10~36.6 | 25~35 | 7 | 0.04 | 0.3 | -- |

Existing researches show that the optimal growth temperature for the vegetative cell is 15-25 °C, the optimal light intensity is 30-50 $\mu$mol $m^{-2}s^{-1}$, the optimal pH is neutral to slightly alkaline, and the carbon source NaAc can be used for mixotrophic growth although *Haematococcus pluvialis* varies from strain to strain. High illumination, high temperature, nutrient (N,P) deprivation, salt stress (NaCl, NaAc, etc.), and oxidative stress (active oxygen, oxygen free radicals and dissolved oxygen) and many other conditions can induce the accumulation of intracellular astaxanthin, and they are collectively known as induction conditions or stress conditions inhibiting cell growth and division and having a synergy effect.

The traditional two-stage photoautotrophic cultivation systems cannot overcome some obstacles, such as pollution by other microorganism, low yield, influence by seasonal cycle, low rate of land utilization, and high cost.

The final cell density of photoautotrophic cultivation is not high, because it is difficult to keep the vegetative stage for a long time due to the rigid requirements for physical conditions under which *Haematococcus pluvialis* is cultivated. The weight of cyst cells can slowly increase, but the vegetative reproduction no longer carries on, and the cell number cannot increase rapidly. Accordingly, the maximum number of cells in the photoautotrophic cultivation is limited. *Haematococcus pluvialis* is very sensitive to changes in environment. The log phase of growth period is short. Furthermore, their ability to inhibit bacteria and protozoa pollution is very poor during the period of vegetative growth, and in extreme circumstances, they lose the ability to reproduce. Thus, it is not easy to establish a stable and efficient cultivation technical system. Therefore, in the cultivation of *Haematococcus pluvialis* for astaxanthin production, it is difficult to select the strains, design the optical bioreactor, control the high cell density culture conditions, and control the astaxanthin accumulation.

At present, two-stage production mode has been successfully adopted. In the first stage, a closed photo bioreactor cultivation system is adopted to realize high-density vegetative growth so as to overcome the problem of pollution.

Then, a conventional open pool system is adopted under the condition of stress to accumulate astaxanthin. Currently in the world, only several large companies such as Cyanotech and Aquasearch can achieve the successful production in industry scale.

In spite that *Haematococcus pluvialis* can survive under a wide range of temperature, the cell growth and the astaxanthin accumulation are carried out under different temperatures. It has been reported that the temperature of 15-25 °C is relatively appropriate for the cell growth, while the temperature of 25-35 °C is more ideal for the astaxanthin accumulation.

One reason why microalgae can be cultivated to produce astaxanthin in industry scale in minority areas, such as Hawaii, the United States is because Hawaii is located in the tropics where the light is enough and the temperature is relatively high. This temperature is very suitable for the astaxanthin accumulation. Although the higher temperature is not conducive to the fast growth of *Haematococcus pluvialis*, there is a special geographic condition in the area: cold water can be easily obtained for cooling. Thus, the control of temperature to grow *Haematococcus pluvialis* is not a problem. In fact, those companies like Cyanotech and Aquasearch use cold water from the sea at depth of (600 m) to control the temperature economically and effectively. So far, this method is restricted by geographical condition, and thus is not suitable for other places like China.

The growth conditions for *Haematococcus pluvialis* are relatively mild, and thus a lot of harmful biology like rotifers, protozoa and other microalgae can grow in the cultivation medium. The prevention of biological pollution becomes a problem which is difficult to overcome in the large scale microalgae cultivation. Early experiments showed that in an open pond cultivation process, about 4-5days after inoculation, rotifers which can devour *Haematococcus pluvialis* appear, resulting in the failure of the whole cultivation. If the microalgal cells can be tranformed to cyst, their ability to inhibit harmful biology is enhanced greatly.

The growth rate of *Haematococcus pluvialis* is low, *Haematococcus pluvialis* is vulnerable to pollution, and the suitable growth temperature of *Haematococcus pluvialis* is low. These characteristics limit the high-density and large-scale cultivation of *Haematococcus pluvialis.* For the mass production of microalgal cells, closed photo-bioreactors are widely used. It has been reported that closed bioreactors are used for the growth of *Haematococcus pluvialis*. There are three types of the closed photo-bioreactors: column type, plate type and tube type. The research focus has been transferred from the application of reactor to the improved structure and operation parameters thereof (ventilation rate, mass transfer rate, etc.). However, it is difficult to control the temperature and light intensity, clean and magnify the reactor, besides a series of existing problems such as high maintenance costs. Therefore, it is necessary to use the existing mature fermentation industry equipment for the high density cultivation of *Haematiococcus pluvialis*.

On the other hand, although heterotrophic cultivation of microalgae has disadvantages of low intracellular astaxanthin content and low chlorophyll pigment content, the microalgae of high cell density can be heterotrophically cultivated at the end of fermentation and grow more quickly in the heterotrophic culture. Heterotrophic cultivation can obtain high cell density and cell growth rate. The maximum cell dry weight reported in literatures was 7 g/L (Hata N, Ogbonna JC, Hasegawa Y, Taroda H, Tanaka H: Production of astaxanthin by Haematococcus pluvialis in a sequential heterotrophic-photoautotrophic culture. Journal of Applied Phycology 2001, 13(5):395-402), the cell production rate was 0.3 g/L/d, the production rate of astaxanthin was low (only 4.4mg/L/d), and the astaxanthin content can be 1.85% after 8-day photo induction. The heterotrophic cultivation was carried out in a 2.3L fermenter, while the photoautotrophic cultivation was carried out in a glass vessel indoors (diameter: 16 cm, liquid loaded: 900 ml, depth: 5.5 cm). The light was set on the top of the vessel, and the light intensity on the liquid surface was 950 $\mu$mol $m^{-2}s^{-1}$. The temperature was maintained at 30 °C. The stirring was realized by a magnetic stirrer (100rpm), and air containing 5% $CO_2$ was aerated into the culture. Although the method applied heterotrophic- photoautotrophic mode, there are four defects:

1) The literatures studied fed-batch cultivation and repeated fed-batch cultivation. The maximum cell dry weight of 7 g/L was obtained in the fed-batch cultivation. However, the demurrage phase in the heterotrophic cultivation is long, and the average cell growth rate is low (about 0.3 g/L/d).
2) In order to prevent bacteria breeding in the photoautotrophic stage caused by organic matter (sodium acetate) which was used in the heterotrophic fermentation as carbon source, it is necessary to stop the feeding of the nutrient into the culture so as to deplete the carbon source before high photo-induction, without considering the contents of nitrate and phosphorus. Therefore, the cells cannot survive well under the stress conditions and the rate of astaxanthin accumulation is low.
3) In the heterotrophic stage, the basal medium was used but no plant growth hormone-like chemicals were added to promote the growth. During the whole process, the pH value was maintained at 7.5-8.0 by intermittent flow with the un-optimized feeding medium. This method did not consider the differences between heterotrophic cultivation and photoautotrophic cultivation in terms of nutritional needs, resulting in poor growth performance and low cell growth. In addition, the intermittent feeding caused variable pH values and fluctuant concentration of each element in the medium (such as improper feeding of medium elements, resulting in lack of nitrogen, phosphorus and magnesium at the end of cultivation). This will easily cause adverse effects on the cell growth. Especially, in order to

add the above mentioned elements, there is a need to open the tank for additional loading operation, which increases the risk of bacteria contamination.

4) When the heterotrophic stage was transferred to photoautotrophic stage, no medium was added into the culture of the microalgae and the culture was not diluted. This will cause the problems as follows: 1) a large quantity of microalgal cells were dead, because the microalgal cells without dilution to a lower density are maintained at a density of 5.5 g/L which is the value at the end of heterotrophic culture. With this high-density, as a result of self-shading effects, a large number of cells cannot get sufficient light to survive, so that the microalgal cells died (the number of initial inoculation of 650000 cells/ml was reduced to only 210000 cells/ml in the end, namely, 70% cells were lost; 2) intracellular astaxanthin content increases slowly, because the culture was not diluted before transferred for photoautotrophic cultivation. However, the nutrient requirements for the heterotrophic cultivation and photoautotrophic of *Haematococcus pluvialis* are different, so the astaxanthin content increases relatively more slowly. After 8-day photo-induction, the astaxanthin content can only be increased from 0.57% to 1.85%.

It can be concluded that the maximum cell dry weight reported in literatures was 7 g/L, the cell average growth rate was 0.3 g/L/d; the astaxanthin production rate was low (4.4 mg/L/d), the astaxanthin content was not high (after 8-day photo-induction, the astaxanthin content was only 1.85%). The method has no advantages compared to the traditional photoautotrophic two-stage cultivation for astaxanthin production. Therefore, it is necessary to find an efficient cultivation method for astaxanthin production by using microalgae.

Besides heterotrophic and photoautotrophic cultivation of microalgae, there is another mode that is not commonly used in the art, i.e. mixotrophic cultivation. However, this mode needs a sterile closed photobioreactor. On the other hand, the light penetration prevents the magnification of the photobioreactor, so it is not applicable in the large scale usage.

It can be concluded that both photoautotrophic cultivation and heterotrophic cultivation have low content of intracellular astaxanthin and low astaxanthin production rate, and high costs of microalgae large-scale cultivation and these disadvantages have restricted the application of microalgae cultivation in the industrialization and production of astaxanthin. Therefore, it is necessary to explore a new method for microalgae cultivation process to significantly increase the production rate of astaxanthin and the astaxanthin content, while substantially decreasing the costs of microalgae large-scale cultivation, so as to meet the wide requirements for mass production of astaxanthin.

Considering the advantages and disadvantages of the above mentioned modes, the present invention develops a mode named "sequential heterotrophic-dilution-photoinduction" cultivation mode, comprising the steps as follows: (1) heterotrophically cultivating a microalgae which can produce astaxanthin in a bioreactor to obtain microalgal cells with high density; (2) timely diluting the culture of the microalgae with a medium containing no organic carbon source when the organic carbon source and nitrogen source in the culture are nearly depleted; (3) photo-inducing the culture to quickly accumulate the intracellular astaxanthin. Heterotrophic stage of the mode is carried out in a shake flask, or a mechanical agitator, airlift, or bubbling bioreactor to obtain the microalgal cells with high density in a short period of time. Photo-induction can be applied in any system used for the photoautotrophic cultivation of microalgae to increase the astaxanthin content in the microalgal cells. Heterotrophic and photo-induction stages are conducted separately and independently. According to the cell density and the nutrients in the culture and the outdoor light intensity, the culture of the microalgae released from the heterotrophic stage is diluted with a photo-induction medium and then transferred to the photo-induction stage. The dilution step can provide adequate illumination for the photo-induction and also maintain the nutrient content in the culture at a low level to provide stress conditions for the astaxanthin accumulation, so as to rapidly increase the intracellular astaxanthin content.

The present invention divides the cultivation of microalgae for astaxanthin production into three stages: heterotrophic cultivation stage for microalgal growth to quickly obtain the cells with high density, dilution stage to reduce the microalgal cell density and provide nutrient stress, and photo-induction stage to increase the intracellular astaxanthin content and further improve the quantity of microalgal cells. A lot of microalgal cells suitable for astaxanthin accumulation can be obtained in a short time by heterotrophic cultivation. The culture of the microalgae is transferred to photo-induction stage after the dilution stage. The astaxanthin content in the cells can be quickly increased up to several times of the initial content. The present invention has the following advantages:

(1) Heterotrophic cultivation can maintain the cells of *Haematococcus pluvialis* in vegetative form for a long time and obtain a high cell production rate. In one embodiment, the average cell production rate is 1.53 g/L/d, up to 5.74 g/L/d. The microalgal cell density at the end of the heterotrophic cultivation can be as high as 26.01 g/L, so the photo-induced microalgal cell density can be very high (2-10 g/L), which is 5-10 times of that in the conventional photoautotrophic cultivation (about 0.2-2 g/L).

(2) The microalgal cells released from the heterotrophic stage can be directly photo-induced under the stress conditions to accumulate astaxanthin just after the dilution of the culture with the photo-induction medium. There is no need of adaptation or transition, and thus the period for photo-induction can be short (about 5 to 7 days), compared

to that for traditional photoautotrophic cultivation mode (about 14 to 30 days). The cell dry weight in the culture at the end of the photo-induction is increased during both the photo-induction stage of the present invention and the astaxanthin accumulation stage of the traditional photoautotrophic cultivation. Therefore, the volumetric production rate of astaxanthin per unit of culture at this stage can be enhanced more than several times than that of traditional photoautotrophic cultivation.

(3) Compared to the photoautotrophic cultivation of microalgae, the photo-induction requires smaller areas due to the high photo-induced microalgal cell density (i.e. the areal production rate of astaxanthin is very high). The high cell density also reduces the recovery cost greatly.

(4) The heterotrophic cultivation is almost not affected by climate and weather, and the photo-induction can be carried out in greenhouse. The light source can be natural sunlight or artificial light. The photo-induction can be conducted in a wide range of temperate (15-35 °C). High temperature can promote astaxanthin accumulation. Although low temperature cannot promote astaxanthin accumulation, the quick astaxanthin accumulation can be realized by artificial heating-up, high salt, high carbon/nitrogen ratio, high light intensity and other collaborative stress conditions because the photo-induction area is small. Therefore, the method of the present invention can be applied in the large-scale continuous production of astaxanthin.

(5) The majority of the cells are in the form of spore at the end of heterotrophic cultivation. Their resistance is stronger than vegetative cells. The diluted culture still has a high density and has an advantage of population. Therefore, the culture during the photo-induction is not susceptible to biological pollution such as protozoa and other harmful biology. The biomass loss due to pollution like protozoa and severe stress which are common in the traditional two-stage photoautotrophic cultivation can be minimized in the present invention.

To sum up, the "sequential heterotrophic-dilution-photoinduction" cultivation mode of the present invention reasonably combines the advantages of the heterotrophic cultivation and the photo-induction cultivation. Compared to the other modes, it has advantages of higher production rate, more flexible integrated culture system, and lower production costs. These advantages can fully utilize heterotrophic cultivation mode to obtain the culture of the microalgae with high density and the quick astaxanthin accumulation during the photo-induction phase, providing an important technical solution to address the problem in the large scale production of astaxanthin from microalgal.

Through analysis of related patents and patent applications, it can be concluded that the existing patents and patent applications mainly focus on photo-bioreactors and devices for cultivation, mediums for photoautotrophic cultivation of *Haematococcus pluvialis,* and new methods for extraction of astaxanthin etc. The cultivation is mainly related to photoautotrophic cultivation. However, no documents are related to the method for "sequential-heterotrophic-dilution-photoinduction" cultivation of microalge e.g. *Haematococcus pluvialis*, *Chlorella zofingiensis* which are suitable for astaxanthin production.

## SUMMARY OF THE INVENTION

The present invention provides a new method for "sequential heterotrophic-dilution-photoinduction" cultivation to rapidly accumulate astaxanthin in a microalgae, comprising: heterotrophically cultivating the microalgae, obtaining and diluting a heterotrophic culture of the microalgae, and photo-inducing the culture.

On the other hand, the present invention provides a method for quickly increasing astaxanthin content in a microalgae, comprising heterotrophically cultivating the microalgae and photo-inducing a heterotrophic culture of the microalgae after dilution.

The present invention also provides a method for producing astaxanthin, comprising: heterotrophically cultivating a microalgae, photo-inducing a heterotrophic culture of the microalgae after dilution, collecting microalgal cells, and extracting the astaxanthin.

In the method according to the present invention, the microalgal cells obtained from the heterotrophic cultivation can be directly photo-induced.

The method according to the present invention can rapidly accumulate intracellular astaxanthin, significantly improve the production rate, reduce the production costs, and provide astaxanthin with high quality.

In the method according to the present invention, the pH and the content of carbon, nitrogen, and phosphorus is controlled, by feeding, within a given range during the heterotrophic cultivation, and the content of carbon, nitrogen and phosphorus is very low or even depleted after the heterotrophic cultivation.

In one embodiment, the content of carbon, nitrogen and phosphorus in a heterotrophic medium is completely depleted after the heterotrophic cultivation.

In one embodiment, the pH value of the culture is controlled at a constant value within the range between 4.0 and 10.0 such as pH 7.5 by feeding during the heterotrophic cultivation. In a preferred embodiment, the pH value of the culture is controlled within the range between 5.0 and 9.0, more preferably between 7.0 and 8.0.

It should be understood that, a small variation of the pH value is allowed. For example, a±Y variation of the

pH value is allowed, wherein Y≤1.0, e.g. Y≦0.2, Y≦ 0.1. In some cases, Y=0. Therefore, in one embodiment, the pH value of the culture is controlled at X±Y by feeding, wherein 5.0≤X±Y≤9.0. For example, in one embodiment, the pH value of the culture is controlled at 7.5±0.3 by feeding.

During the heterotrophic cultivation of the microalgae, the content of carbon, nitrogen, and/or phosphorus is controlled, by feeding, within a given range. For example, the carbon content in the culture is controlled within the range between 0.5 and 50 mM, the nitrogen content in the culture is controlled within the range between 0.5 and 10 mM, and the phosphorus content in the culture is controlled within the range between 0.01 and 0.5 mM.

In one embodiment, the content of carbon, nitrogen, and phosphorus is controlled, by feeding, within a given range. For example, the carbon content in the culture is controlled within the range between 0.5 and 50 mM, the nitrogen content in the culture is controlled within the range between 0.5 and 10 mM, and the phosphorus content in the culture is controlled within the range between 0.01 and 0.5 mM.

In one embodiment, the magnesium content is controlled within the range between 0.00001 and 0.001 mM by feeding.

In one embodiment, the microalgae is selected from *Haematococcus pluvialis* or Chlorella (*Chlorella zofingiensis*), etc.

In one embodiment, the microalgae is heterotrophically cultivated by the following steps: adding the heterotrophic medium at pH 4.0-10.0 into a bioreactor and adding microalgal seeds of 0.1-50% working volume into the bioreactor for batch, feed-batch, repeated feed-batch, semi-continuous and continuous cultivation at culture temperature of 10-40 °C, at pH value lower than 10.0, and with dissolved oxygen more than 0.1%..

In one embodiment, the heterotrophic culture of the microalgae is diluted by the photo-induction medium to reduce the microalgal cell density to 0.1-20g/L and is maintained at pH 4.0-9.0.

In one embodiment, the diluted culture the microalgae is photo-induced in a photo-induction device, at photo-induction temperature of 5-50 °C, with continuous or intermittent light intensity of 0.1-150klx, and for 1-480 hours.

In one embodiment, the heterotrophic medium contains nitrogen source, organic carbon source, a small amount of inorganic salt, plant growth hormone, trace elements and water, or consists thereof; the photo-induction medium contains plant growth hormone, nitrogen source, inorganic salts and water, or consists thereof.

In one embodiment, when the microalgae is selected from *Haematococcus pluvialis,* the heterotrophic medium used herein substantially consists of the following components: sodium acetate 0.1-5.0 g/L, $NaNO_3$ 0.05-1.5 g/L, $CaCl_2 \cdot 7H_2O$ 0.05-1.5 g/L, $KH_2PO_4$ 0.01-1.5 g/L, $MgSO_4 \cdot 7\,H_2O$ 0.01-1.0 g/L, $FeSO_4 \cdot 7\,H_2O$ 0.01-0.05 g/L, plant growth hormone 0.001-35 mg/L, trace element 0.5-4 mL and water.

In one embodiment, the microalgae is heterotrophically cultivated in a shake flask, or an agitator, airlift or bubbling bioreactor. The photo-induction is carried out in a shake flask, an open raceway or circle pond, a closed flat plate type, a pipeline type, a column type photo-bioreactor, a film bag, a hanging bag, or any other photoautotrophic cultivation devices. The light source for the photo-induction can be natural daylight or artificial light.

In one embodiment, the astaxanthin is extracted by supercritical $CO_2$ extraction method, organic solvent extraction method, or ultrasonic-assisted solvent extraction method.

In one embodiment, the method according to the present invention further comprises: solid/liquid separating the photo-induced microalgal cells (i.e. for collection) and then drying the microalgal cells to obtain powders containing the astaxanthin.

In one embodiment, the method according to the present invention further comprises: mixing the microalgae after astaxanthin extraction and other pigments to form dry powders, or extracting other bioactive substances from the microalgal cells.

In one embodiment, the other pigments include chlorophyll. In one embodiment, the bioactive substances include protein, lipids, chlorophyll and polysaccharide.

## DESCRIPTION OF THE DRAWINGS

Figure 1 shows the time course of heterotrophic cultivation in a 5 L fermenter (including the optimal heterotrophic growth process of the present invention, the heterotrophic growth process with only pH control but without optimization of initial and feeding medium, and the heterotrophic growth process with pH control and optimization of the initial and medium feeding, but without plant growth hormone).

Figure 2 shows the photo-induction process of *Haematococcus pluvialis* in an outdoor 2 L column bioreactor when the carbon, nitrogen, phosphorus nutrients are completely depleted.

Figure 3 shows the photo-induction process of *Haematococcus pluvialis* in an outdoor 2 L column bioreactor when

the carbon, nitrogen, phosphorus nutrients are not depleted.

## DESCRIPTION OF THE PREFERRED EMBODIMENT

Microalgae used in the method according to the present invention includes those suitable for synthesis of astaxanthin and heterotrophic cultivation, including but not limited to *Haematococcus pluvialis,* Chlorella (*Chlorella zofingiensis*), etc. In a preferred embodiment, this present invention uses *Haematococcus pluvialis* to produce astaxanthin.

Plant growth hormone used in the medium according to the present invention (including heterotrophic medium and photo-induction medium) includes but not limited to 2, 4-dichlorobenzene oxygen ethanoic acid, benzyl amino purine, exogenous gibberellin, 3-indole butyric acid, naphthalene acetic acid and canola, etc. The medium can contain one or more than one plant growth hormone. Total content of plant growth hormone in the medium can be 0.001-35 mg/L medium, generally 0.001-20 mg/L, more generally 0.001-15 mg/L, 0.005-10 mg/L, 0.01-10 mg/L, or 0.1-5 mg/L.

In one embodiment, the plant growth hormone contains, if any, 2, 4-dichlorobenzene oxygen ethanoic acid 0.001-5 mg/L, benzyl amino purine 0.001-5 mg/L, exogenous gibberellin 0.001-5 mg/L, 3-indolebutyric acid 0.001-5 mg/L, naphthalene acetic acid 0.001-5 mg/L, canola 0.001-5 mg/L. Preferably, their concentrations respectively are 0.01-4 mg/L, 0.1-4 mg/L, 0.3-4 mg/L, 0.3-3 mg/L, 0.5-2.5 mg/L.

The above mentioned plant growth hormones are available in the market and then are added directly to the medium either for heterotrophic cultivation or for photo-induction to synthesize astaxanthin. Examples of the mediums will be described below.

1. Heterotrophic cultivation of microalgae with high density in a bioreactor

This step is aimed to quickly obtain a large quantity of microalgal cells for astaxanthin accumulation during the photo-induction.

Various well known mediums with organic carbon source (e.g. sodium acetate) can be used for the heterotrophic cultivation of the microalgae. Usually, the heterotrophic medium according to the present invention contains nitrogen source, organic carbon source, plant growth hormone, a small amount of inorganic salt, trace elements and water.

This medium includes C medium (Ichimura, T. 1971 Sexual cell division and conjugation-papilla formation in sexual reproduction of Closterium strigosum. In Proceedings of the Seventh International Seaweed Symposium, University of Tokyo Press, Tokyo, p. 208-214.), MCM medium (Borowitzka et al., 1991), BG-11 medium (Boussiba and Vonshak, 1991), BBM medium (Nichols and Bold, 1969), BAR medium (Barbera et al., 1993), KM medium (Kobayashi et al., 1991), Z8 medium (Renstrom et al., 1981), A9 medium (Lee and Pirt, 1981), OHM medium (Fa'bregas et al., 2000), KM1 medium (Usha et al., 1999, Garc'ia-Malea et al., 2005), HK2 medium (Chen et al., 1997), HK3 medium (Gong and Chen, 1998) etc.

C medium used in the present invention substantially consists of $KNO_3$, $CaNO_3$, sodium acetate, a small amount of inorganic salt, trace elements and water, in addition to some plant growth hormones.

The term "substantially consists of......" means that besides the main components e.g. $KNO_3$, $CaNO_3$, sodium acetate, a small amount of inorganic salt, trace elements, and water, there are also other elements which have no substantial effects on the basic or new features of the composition (namely, to maintain microalgae at a high cell density in a short cultivation cycle and greatly improve the content of active substances compared to the conventional heterotrophic cultivation). The term "consists of......" means that the composition only includes the specific components without other components, except impurities with an acceptable content.

In the medium, the components in the medium may vary within a certain range but without substantially affecting the microalgal cell density and the microalgal cell quality. Accordingly, the amount of these components should not be restricted by the examples. As known by one skilled in the art, a small amount of inorganic salts e.g. magnesium sulfate, calcium chloride, ferrous sulfate and phosphate, etc., a small amount of trace elements e.g. Mn, Zn, B, I, M, Cu, Co etc., and plant growth hormones, including single hormone or combination of various hormones can be added to the medium,.

In the present invention, the trace element is selected from one or more than one of $H_3BO_3$, $ZnSO_4 \cdot 7\ H_2O$, $MnCl_2 \cdot H_2O$, $(NH_4)_6Mo_7O_{24} \cdot 4\ H_2O$, $CuSO_4 \cdot 5\ H_2O$ and $Co(NO_3)_2 \cdot 6\ H_2O$. The dosage of inorganic salts and trace elements can be determined according to the common knowledge.

In one embodiment, the heterotrophic medium according to the present invention substantially consists of the following components:

In one embodiment, when the microalgae is selected from *Haematococcus pluvialis,* the heterotrophic medium used herein substantially consists of the following components: sodium acetate 0.1-5.0 g/L, $NaNO_3$ 0.05-1.5 g/L, $CaCl_2 \cdot 7H_2O$ 0.05-1.5 g/L, $KH_2PO_4$ 0.01-1.5 g/L, $MgSO_4 \cdot 7\ H_2O$ 0.01-1.0 g/L, $FeSO_4 \cdot 7\ H_2O$ 0.01-0.05 g/L, plant growth hormone 0.001-35 mg/L, trace element 0.5-4 mL and water.

In one embodiment, the plant growth hormone in the heterotrophic medium contains 2, 4-dichlorobenzene oxygen ethanoic acid 0.001-5 mg/L, benzyl amino purine 0.001-5 mg/L, exogenous gibberellin 0.001-5 mg/L, 3-indolebutyric acid 0.001-5 mg/L, naphthalene acetic acid 0.001-5 mg/L, canola 0.001-5 mg/L.

In one embodiment, the plant growth hormone in heterotrophic medium contains benzyl amino purine 0.001-5 mg/L and 3-indolebutyric acid 0.001-5 mg/L.

After the preparation of medium in accordance with the above formula, one skilled in the art may use the common technologies such as by adding acid or base to adjust the medium to pH 4.0-10.0, and may sterilize the medium under 115-125 °C for 15-30 minutes. Batch, feed-batch, semi-continuous and continuous cultivation can be carried out in the heterotrophic cultivation.

When the feed-batch is carried out in the heterotrophic cultivation, the bioreactor is filled with the prepared medium and water is added to the work volume, usually with a load coefficient of 0.6-0.8. The medium is then sterilized at 121 °C for about 20 minutes. When the temperature decreases to 20-35 °C, microalgal seeds of 0.1-50% working volume is added for the heterotrophic cultivation.

During the heterotrophic cultivation, the pH value is maintained within a given range, such as between 7.0 and 8.0, by controlling the continuous flow rate of feeding medium. In a preferred embodiment, the pH is controlled at 7.5.

The feeding medium includes nutritive salts, such as organic carbon source (e.g. sodium acetate), nitrogen source (e.g. $CaNO_3$, $KNO_3$), plant growth hormone and inorganic salt. These added nutritive salts are concentrated in the above-mentioned corresponding medium to prompt the growth of microalgae. Organic carbon source, nitrogen source, plant growth hormone and inorganic salt are added into the feeding medium in the way that the concentrations of the corresponding components in the culture are equivalent or similar to the initial concentrations when the heterotrophic cultivation is started so as to promote the growth of microalgae. One skilled in the art can also adjust the corresponding components according to the actual growing condition of microalgae, for example, by increasing or decreasing the concentration of some components so as to promote the growth of microalgae.

When adding the nutritive salts, the content of carbon, nitrogen and/or phosphorus should be timely monitored in order to adjust the content of these materials in the feeding medium appropriately. The carbon content is controlled within the range of 0.5-50 mM (normally 1.0-40 mM, 1.0-30 mM, 1.0-20 mM, 1.0-10 mM), the nitrogen content is controlled within the range of 0.5-10 mM (normally 0.5-8 mM, 0.5-6 mM, 1.0-6 mM, 1.0-5.0 mM), the phosphorus content is controlled within the range of 0.01-0.5 mM (normally 0.01-0.4 mM, 0.05-0.3 mM, 0.05-0.2 mM, 0.05-0.1 mM) so as to ensure the concentration stability of these materials. Preferably, the magnesium content in the culture of the microalgae is monitored and controlled within the range of 0.00001-0.001 mM by feeding (normally 0.00001-0.0008 mM, 0.00003-0.0005 mM).

When the microalgal cell density reaches the required value at a certain stage, the control conditions are adjusted to substantially deplete carbon, nitrogen and/or phosphorus. The heterotrophic cultivation stage concludes. Normally, when heterotrophic cultivation stage concludes, the contents of carbon, nitrogen and/or phosphorus are very low in the culture. For example, the contents of carbon and nitrogen are lower than 0.1 mM, 0.05 mM, 0.01 mM or lower, or even are 0; the phosphorus content is lower than 0.005 mM, 0.003 mM, 0.001 mM or lower, or even are 0.

No matter what kind of cultivation is used, the cultivation conditions must be strictly controlled for the normal growth of microalgae during the cultivation. Normally, the temperature is controlled at 20-35 °C, for example, 25-30 °C, the dissolved oxygen is controlled not less than 5% of air saturation concentration by adjusting the aeration and agitation, and the pH value is controlled not higher than 9.0. In a preferred embodiment, the dissolved oxygen is not less than 10% but not more than 30% of air saturation concentration, the pH value is constantly controlled at 7.5-8.0, the aeration amount is less than 0.3 VVM, and the agitation rate is less than 200 rpm.

The heterotrophic cultivation can be conducted in bioreactors e.g. shake flask, mechanical agitator, air lift and bubbling bioreactors.

2. Dilution of the culture of the microalgae with high density

This step is aimed to reduce the microalgal cell density so as to enable the microalgae for production of astaxanthin to absorb light energy efficiently during the photo-induction and improve the utilization efficiency of light energy. This step is also aimed to adjust the nutrients in the photo-induction medium for nutritional stress so as to rapidly accumulate the astaxanthin.

The culture of the microalgae with high density acquired from the heterotrophic cultivation should be diluted by using a dilution medium to maintain the cell density at 0.1-20g/L and maintain the PH value at 4.0-10.0 (when the induction is carried out in an open reactor, it is better not to contain organic carbon source so as to avoid mixed bacteria during the photo-induction stage. However, when the induction is carried out in a closed reactor, the organic carbon source can be contained to increase the cells). In some embodiments, the culture of the microalgae with high density is diluted by water and the medium without organic carbon source to maintain the cell density at 0.1-10g/L and adjust the pH value at 5.0-8.0. In other embodiments, the culture of the microalgae is diluted to maintain the cell density at 1-8g/L and adjust the pH value at 5.0-8.0. In a preferred embodiment, the cell density is maintained at 1.0-5.0g/L and the pH value is adjusted at 5.0-8.0 by adding $CO_2$.

Various well known mediums can be used for the dilution of the culture. Usually, the photo-induction medium

contains nitrogen source, plant growth hormone, inorganic salt, and water, or consists thereof. Compared to the heterotrophic medium, the photo-induction medium contains no or few organic carbon source. $CO_2$ can be added during the cultivation.

In a preferred embodiment, the microalgal cells with high density acquired from the heterotrophic cultivation is diluted by an initial medium without organic carbon source and lacking of nitrogen and phosphorus.

In one embodiment, the dilution medium (i.e. photo-induction medium) contains: $MgSO_4 \cdot 7H_2O$ 0.01~0.1 g/L, $NaH_2PO_4$ 0.01~0.1 g/L, KCl 0.1-1 g/L, $CaCl_2$ 0.01~0.2 g/L, $FeSO_4 \cdot 7H_2O$ 0.01~0.06 g/L, EDTA 0.020-0.052g/L and plant growth hormone 0.001-35 mg/L.

In one embodiment, the plant growth hormone in the dilution medium (i.e. photo-induction medium) contains: 2, 4-dichlorobenzene oxygen ethanoic acid 0.001-5 mg/L, benzyl amino purine 0.001-5 mg/L, exogenous gibberellin 0.001-5 mg/L, 3-indolebutyric acid 0.001-5 mg/L, naphthalene acetic acid 0.001-5 mg/L and/or canola 0.001-5 mg/L.

In one embodiment, the plant growth hormone in the dilution medium (i.e. photo-induction medium) contains: benzyl amino purine 0.001-5 mg/L and 3-indolebutyric acid 0.001-5 mg/L.

The dilution medium needs no autoclave sterilization and can be used after its pH value is adjusted to 5.0-9.0.

It should be understood that in some embodiments, the microalgal cells acquired by from the heterotrophic cultivation can be directly photo-induced without dilution. This depends on the rational matching among the heterotrophic cultivation density, the components of the heterotrophic medium and the actual induction conditions (such as light intensity, temperature, etc.).

3. Photo-induction

This step is aimed to expose the microalgae which can produce astaxanthin to sufficient lights, rapidly synthesize and accumulate a large amount of astaxanthin by photo-induction, and appropriately increase the microalgal cell density in the culture.

As mentioned above, after diluting the culture of the microalgae with high density, the culture is transferred into a photo-induction device for photo-induction or the microalgal cells are photo-induced on a solid membrane surface by using a semisolid adherent method, at photo-induction temperature of 5-50 °C, with continuous or intermittent light intensity of 0.1-150 klx for 1-480 hours, and with ventilation capacity of 0.1- 2.0 vvm. The photo-bioreactor includes all closed photo-bioreactor (shake flask, pipeline type, plate type, column type, film bag and hanging bag etc.) and all open photo-bioreactor (raceway pond, circle pond, and bubbling basin etc.).

Usually, the cultivation temperature is controlled within the range of 15-35 °C, e.g. 18-35 °C, 20-35 °C and 20-30 °C etc. Usually, the light intensity is controlled within the range of 1-70 klx, e.g. 1-60 klx, 1-50 klx, 1-40 klx, 1-30 klx, 1-20 klx, 1-10 klx etc, which can depend on the specific production conditions. Usually, if gas is introduced to thoroughly mix the culture of the microalgae, the ventilation capacity can be controlled within the range of 0.1-2.0 vvm, e.g. 0.2~1.8 vvm, 0.5~1.5 vvm, 0.8~1.5 vvm, 1.0~1.5 vvm etc. Meanwhile, $CO_2$ with a certain concentration is introduced to provide inorganic carbon source and control the pH value, for example, 0.5%-10% $CO_2$. In another embodiment, the cultivation temperature is controlled within the range of 10~50 °C, the light intensity is controlled within the range of 1-10 klx, and the ventilation capacity is controlled within the range of 0.05-2.0 wm.

In another embodiment, the photo-induction period is 8-480 hours. For example, according to the actual weather conditions, the photo-induction period can be 8-240 hours, 8-120 hours, 8-72 hours, 8-48 hours, 8-24 hours; or the photo-induction period can be 12-72 hours, 12-60 hours, 12-48 hours, 12-36 hours, 12-24 hours or 24-60 hours, 24-48 hours.

The photo-induction medium is selected from modified and improved photoautotrophic medium, including the above mentioned dilution medium. In the present invention, "photo-induction period" includes the whole photo-induction process. For example, the photo-induction period for outdoor cultivation includes the night time without sunlight.

In this application, "illumination time" in the present invention refers to the photo-induction time for microalgae with the above mentioned light intensity, which means the illumination time excludes the night time without sunlight. In some embodiments, the illumination time in the photo-induction step can be 8-120 hours, e.g. 8-72 hours, 8-36 hours, 8-24 hours, 8-18 hours, 8-12 hours, 12-36 hours, 12-24 hours, and any length of time in the above mentioned ranges.

Therefore, the photo-induction step in the present invention also includes photo-induction with illumination time of 8-120 hours. The photo-induction can be conducted by both artificial light and natural daylight.

In one embodiment, when the concentration of astaxanthin in the culture reaches its peak, the photo-induction concludes. Then, the astaxanthin is extracted from the obtained microalgal cells or the microalgal cells are directly collected for the preparation of powders.

4. Collection of microalgal cells, extraction of astaxanthin and comprehensive utilization of microalge.

After the photo-induction, wet microalgal cells are collected through sedimentation or centrifugation. The methods for collecting microalgal cells include but not limited to sedimentation, high-speed centrifugation, flocculation, flotation and filtration etc. The methods for wall-breaking microalgal cells include but are not limited to the wet wall-breaking

methods, e.g. cell autolysis, high pressure homogenization, enzymatic hydrolysis, aqueous phase pyrolysis.

The astaxanthin is extracted from the microalgae by the traditional organic solvent extraction method. First, the organic solvent is added to microalgal slime for extraction. Then, supernatant and microalgal sediment are acquired by stirring and centrifuging. Last, astaxanthin crystals are obtained by concentrating, reducing pressure, stirring and adding water and filtering the supernatant.

Other ingredients in the supernatant, e.g. fatty acid and lutein, can be acquired by gradual separation and extraction. Alternatively, all ingredients in the supernatant are directly mixed with the microalgal sediment and the mixture is spray-dried to obtain microalgal powders.

In a preferred embodiment, the astaxanthin is extracted from the microalgae by supercritical $CO_2$ extraction method. In a more preferred embodiment, the obtained culture of the microalgae is concentrated and then spray-dried to acquire microalgal powders.

In the present invention, the microalgae obtained from the cultivation can be comprehensively utilized to extract polyunsaturated fatty acids, protein, chlorophyll, polysaccharide and other active ingredients. Extraction sequence of active ingredients does not have any special limitation. However, the early extraction step shall not cause the loss of ingredients in the later extraction step.

Microalgal cell dry weight and astaxanthin content in the present invention are determined as follows:

Determination of microalgal cell dry weight: during the cultivation of microalgae, taking V ml culture, centrifuging the culture for 10 minutes at 8000rpm, washing the microalgal cells with deionized water for three times, transferring the microalgal cells into a weighing bottle ($W_1$ (g)), and drying the microalgal cells to a constant weight of $W_2$ (g) in an oven at 105 °C. The dry weight Cx can be calculated according to the following equation:

$$Cx\ (g/L) = (W_2 - W_1)/V/1000$$

Determination of astaxanthin: using the method of high performance liquid chromatography (HPLC), the specific steps of which are described in the following documents: J.P.Yuan, F. Chen, Chromatographic separation and purification of trans-astaxanthin from the extracts of Haematococcus pluvialis, J. Agric. Food Chem. 46 (1998) 3371-3375.

**EXAMPLE**

The following heterotrophic medium and water of are added into a 5 L bioreactor to a volume of 2.5 L, and then sterilized by steam. When the temperature is reduced to 25 °C, *Haematococcus pluvialis* seeds are inoculated for heterotrophic cultivation. The dissolved oxygen is controlled to have not less than 5% of air saturation concentration by adjusting aeration and agitation.

During the heterotrophic cultivation, the pH value is maintained at 7-8 by controlling the rate of continuous flow of feeding medium. The feeding medium includes nutritive salts, such as organic carbon source (e.g. sodium acetate), nitrogen source (e.g. $CaNO_3$, $KNO_3$), inorganic salt and plant growth hormone. These added nutritive salts are concentrated in the above-mentioned corresponding medium to prompt the growth of microalgae. Meanwhile, the content of carbon, nitrogen and/or phosphorus should be timely monitored in order to adjust the content of these materials in the feeding medium appropriately (carbon: 0.5-50 mM, nitrogen: 0.5-10 mM, phosphorus: 0.01-0.5 mM, magnesium: 0.00001-0.001 mM), so as to ensure the concentration stability of these materials. When the microalgal cell density reaches the required value at a certain stage, the control conditions are adjusted to substantially deplete carbon, nitrogen and/or phosphorus. The heterotrophic cultivation stage concludes. In the case that there is no hormone, other operations and experimental conditions are the same, except plant growth hormone in the medium. In the case that there is no optimal control, only the pH value is monitored in the fermented liquid and maintained at 7-8 by using the feeding medium. The content of other materials such as carbon, nitrogen, phosphorus and magnesium is not controlled, and hormonal material also is not added. Other experimental conditions and operations are the same.

The results are shown in Figure 1. At the end of the heterotrophic cultivation, the cell dry weight is 26 g/L when the pH value and the feeding are controlled and plant growth hormone is added; the cell dry weight is 8.7 g/L when the pH value and the feeding are controlled but without adding the plant hormone; the cell dry weight was only about 4.2 g/L when the pH value and the feeding are not controlled and the plant growth hormone is not added. Accordingly, the cell density is increased by 6.2 times compared to the case that the pH value is controlled, the feeding is not controlled controlled and the plant hormone is not added, and is increased by 2.1 times compared to the case that the pH value and the feeding are controlled but without adding the plant hormone.

1L culture of the microalgal obtained from the heterotrophic cultivation is diluted from the density of 8.5 g/L to the density of 1.3 g/L with the photo-induction medium and then photo-induced in an outdoor 2 L column photo-bioreactor at temperature of 28-38 °C, air flow rate of 1 VVM, natural light intensity of about 75 klx each side.

Figure 2 shows the photo-induction process of *Haematococcus pluvialis* in an outdoor 2 L column bioreactor when the carbon, nitrogen, phosphorus nutrients are completely depleted. After a 3-day photo-induction, the cell dry weight reaches 1.92 g/L, astaxanthin content increases from 2.67 mg/gDcw at the beginning of induction stage to 22.56 mg/gDcw at the end (astaxanthin content has increased by about 8.5 times). Based on the 3-days photo-induction, the astaxanthin production rate is 82.24 mg/L/d (which is 3.57 times of the reported highest production rate of 23.04mg/L/d the in photoautotrophic cultivation) (please refer to Figure 2).

Figure 3 shows the photo-induction process of *Haematococcus pluvialis* in an outdoor 2 L column bioreactor when the carbon, nitrogen, phosphorus nutrients are not depleted. After a 3-day photo-induction, the cell dry weight reaches 2.12 g/L, astaxanthin content increases from 2.67 mg/gDcw at the beginning of induction stage to 6.51 mg/gDcw at the end (astaxanthin content has increased by about 2.4 times). Based on the 3-days photo-induction, the astaxanthin production rate is 22.51 mg/L/d (only 27% of the astaxanthin production rate in the case that 3 nutrients i.e. carbon, nitrogen, phosphorus have been totally depleted). Thus, it can be concluded that in the heterotrophic stage, whether carbon, nitrogen and phosphorus are completely depleted is crucial to the increase of astaxanthin production rate.

The heterotrophic medium and the feeding medium contain:

Sodium acetate 0.1-5.0 g/L, $NaNO_3$ 0.05-1.5 g/L, $CaCl_2 \cdot 7H_2O$ 0.05-1.5 g/L, $KH_2PO_4$ 0.01-1.5 g/L, $MgSO_4$ 7 $H_2O$ 0.01-1.0 g/L, $FeSO_4 \cdot 7\ H_2O$ 0.01-0.05 g/L, benzyl amino purine 0.001-5 mg/L, 3-indolebutyric acid 0.001-5 mg/L, trace element 0.5-4 mL and water.

The photo-induction medium contains:

$MgSO_4 \cdot 7\ H_2O$ 0.01-0.1 g/L, $NaH_2PO_4$ 0.01-0.1 g/L, KCl 0.1-1 g/L, $CaCl_2$ 0.01-0.2 g/L, $FeSO_4 \cdot 7\ H_2O$ 0.01-0.06 g/L, EDTA 0.020-0.052 g/L, benzyl amino purine 0.001-5 mg/L, 3-indolebutyric acid 0.001-5 mg/L.

The above description is illustrative and is not restrictive. Many variations of the invention will become apparent to those skilled in the art upon review of the disclosure. The scope of the invention should, therefore, be determined not with reference to the above description, but instead should be determined with reference to the claims along with their full scope or equivalents

## Claims

**1.** A method for producing astaxanthin or increasing astaxanthin content in a microalgae, comprising:

(1) heterotrophically cultivating the microalgae; and
(2) obtaining and diluting a heterotrophic culture of the microalgae and then adding a photo-induction medium into the heterotrophic culture of the microalgae for photo-induction; or directly photo-inducing the obtained heterotrophic culture of the microalgae;

so as to produce astaxanthin or increase astaxanthin content in the microalgae.

**2.** A method for producing astaxanthin, comprising:

(1) heterotrophically cultivating the microalgae, wherein the pH, and content of carbon, nitrogen, and/or phosphorus in a heterotrophic medium is controlled, by feeding, within a given range during the cultivation, and the content of carbon, nitrogen and/or phosphorus in the heterotrophic medium is very low or even depleted after the cultivation;
(2) obtaining and diluting a heterotrophic culture of the microalgae and then adding a photo-induction medium into the heterotrophic culture of the microalgae for photo-induction; or directly photo-inducing the obtained heterotrophic culture of the microalgae; and
(3) collecting microalgal cells and extracting the astaxanthin.

**3.** A method for increasing astaxanthin content in a microalgae, comprising:

(1) heterotrophically cultivating the microalgae, wherein the pH, and content of carbon, nitrogen, and/or phosphorus in a heterotrophic medium is controlled, by feeding, within a given range during the cultivation, and the content of carbon, nitrogen and phosphorus in the heterotrophic medium is very low or even depleted after the cultivation; and
(2) obtaining and diluting a heterotrophic culture of the microalgae and then adding a photo-induction medium into the heterotrophic culture of the microalgae for photo-induction; or directly photo-inducing the obtained heterotrophic culture of the microalgae;
so as to increase astaxanthin content.

4. The method according to Claims 1-3, wherein the microalgae can be cultivated heterotrophically and can produce astaxanthin and is selected from, for example, Haematococcus pluvialis, and Chlorella zofingiensis.

5. The method according to Claims 1-4, wherein the microalgae is heterotrophically cultivated by the following steps: adding the heterotrophic medium at pH 4.0-10.0 into a bioreactor and adding microalgal seeds of 0.1-50% working volume into the bioreactor for batch, feed-batch, repeated feed-batch, semi-continuous and continuous cultivation at culture temperature of 10-40 °C, at pH value lower than 10.0, and with dissolved oxygen more than 0.1%.

6. The method according to Claims 1-5, wherein the heterotrophic culture of the microalgae is diluted by the photo-induction medium or water to reduce the microalgal cell density to 0.1-20g/L and is maintained at pH 4.0-10.0.

7. The method according to Claims 1-6, wherein the diluted culture or the heterotrophic culture of the microalgae is directly photo-induced in a photo-induction device, or the microalgal cells are photo-induced on a solid membrane surface by using a semisolid adherent method, at photo-induction temperature of 5-50 °C, with continuous or intermittent light intensity of 0.1-150klx, and for 1-480 hours.

8. The method according to Claims 1-7, wherein the heterotrophic medium contains nitrogen source, organic carbon source (including but not limited to sodium acetate), plant growth hormone, inorganic salts, trace elements and water, or consists of nitrogen source, organic carbon source (including but not limited to sodium acetate), plant growth hormone, inorganic salts, trace elements and water; the photo-induction medium contains plant growth hormone, nitrogen source, inorganic salts and water, or consists of plant growth hormone, nitrogen source, inorganic salts and water.

9. The method according to Claims 1-8, wherein the microalgae is heterotrophically cultivated in a shake flask, or an agitator, airlift or bubbling bioreactor with internal light or external light;
wherein the photo-induction is carried out in a shake flask, a raceway pond, a circle pond, a flat plate type, pipeline type, column type or spherical photo-bioreactor, a film bag, a hanging bag, or any other photoautotrophic cultivation or photo-induction devices; or the microalgal cells are photo-induced on a solid membrane surface by using a semisolid adherent method; and
wherein light source for the photo-induction can be natural daylight or artificial light.

10. The method according to Claims 1-9, further comprising: solid/liquid separating the photo-induced microalgal cells and then drying the microalgal cells to obtain powders containing the astaxanthin.

11. The method according to Claims 1-10, further comprising: extracting the astaxanthin from the microalgal cells, or mixing the microalgae after astaxanthin extraction and other pigments to form dry powders, or extracting other bioactive substances from the microalgal cells.

12. The method according to Claims 1-11, wherein the astaxanthin is extracted by supercritical $CO_2$ extraction method, organic solvent extraction method, or ultrasonic-assisted solvent extraction method.

13. A heterotrophic medium, containing nitrogen source, organic carbon source (including but not limited to sodium acetate), plant growth hormone, inorganic salts, trace elements and water, or consisting of nitrogen source, organic carbon source (including but not limited to sodium acetate), plant growth hormone, inorganic salts, trace elements and water, for heterotrophically cultivating a microalgae which can be cultivated heterotrophically and can produce astaxanthin.

14. A photo-induction medium, containing plant growth hormone, nitrogen source, inorganic salts and water, or consisting of plant growth hormone, nitrogen source, inorganic salts and water, for heterotrophically cultivating a microalgae which can be cultivated heterotrophically and can produce astaxanthin.

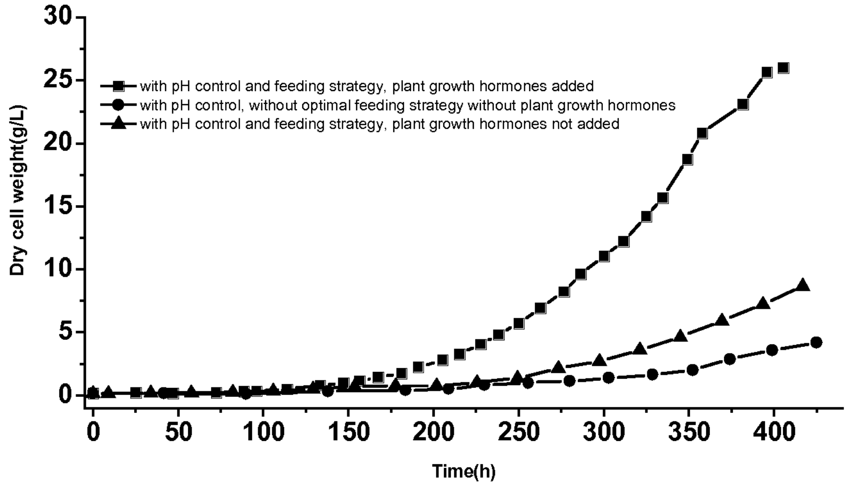


Figure 1

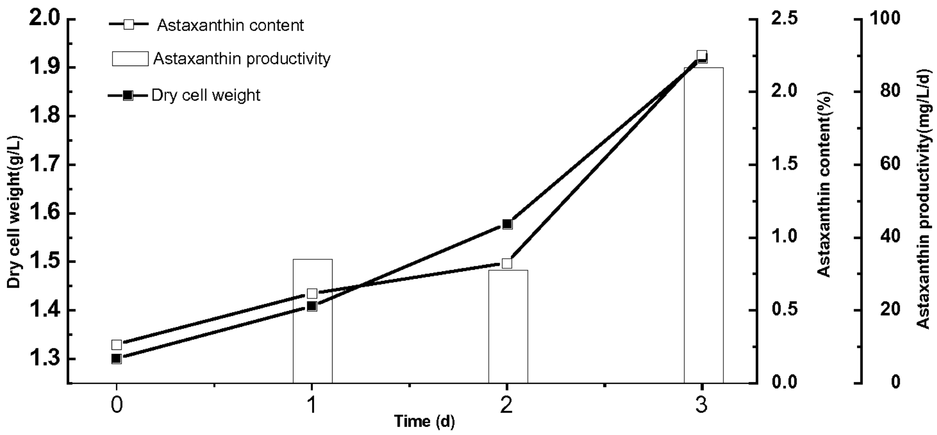


Figure 2

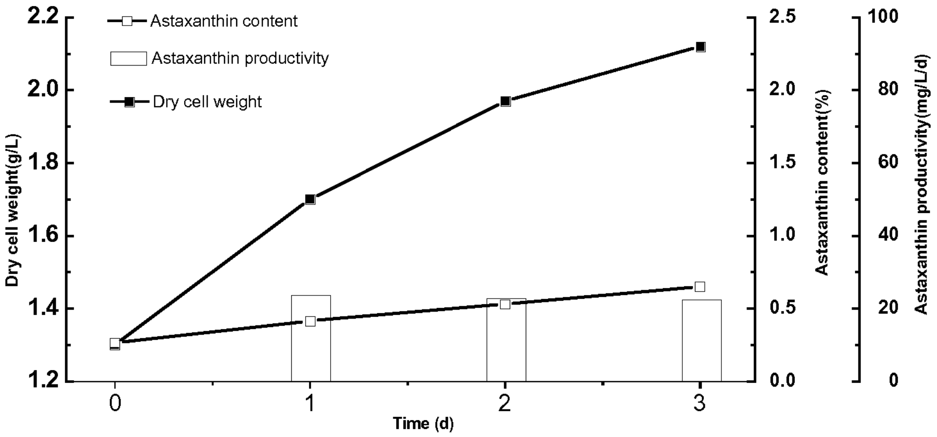
Astaxanthin content
Astaxanthin productivity
Dry cell weight
Dry cell weight(g/L)
2.2
2.0
1.8
1.6
1.4
1.2
Astaxanthin content(%)
2.5
2.0
1.5
1.0
0.5
0.0
Astaxanthin productivity(mg/L/d)
100
80
60
40
20
0
0
1
2
3
Time (d)

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **RANJBAR R ; INOUE R ; SHIRAISHI H ; KATSUDA T ; KATOH S.** High efficiency production of astaxanthin by autotrophic cultivation of Haematococcus pluvialis in a bubble column photobioreactor. *Biochemical Engineering Journal,* 2008, vol. 39 (3), 575-580
- **RANJBAR R ; INOUE R ; KATSUDA T ; YAMAJI H ; KATOH S.** High efficiency production of astaxanthin in an airlift photobioreactor. *Journal of Bioscience and Bioengineering,* 2008, vol. 106 (2), 204-207
- **OLAIZOLA M.** Commercial production of astaxanthin from Haematococcus pluvialis using 25,000-liter outdoor photobioreactors. *Journal of Applied Phycology,* 2000, vol. 12 (3), 499-506
- **GARCIA-MALEA MC ; ACIÉN FG ; FERNÁNDEZ JM ; CERÓN MC ; MOLINA E.** Continuous production of green cells of Haematococcus pluvialis: Modeling of the irradiance effect. *Enzyme and Microbial Technology,* 2006, vol. 38 (7), 981-989
- **HATA N ; OGBONNA JC ; HASEGAWA Y ; TARODA H ; TANAKA H.** Production of astaxanthin by Haematococcus pluvialis in a sequential heterotrophic-photoautotrophic culture. *Journal of Applied Phycology,* 2001, vol. 13 (5), 395-402
- Sexual cell division and conjugation-papilla formation in sexual reproduction of Closterium strigosum. **ICHIMURA, T.** Proceedings of the Seventh International Seaweed Symposium. University of Tokyo Press, 1971, 208-214
- **J.P.YUAN ; F. CHEN.** Chromatographic separation and purification of trans-astaxanthin from the extracts of Haematococcus pluvialis. *J. Agric. Food Chem.,* 1998, vol. 46, 3371-3375